# EUROPEAN PATENT APPLICATION

(11) **EP 4 445 869 A1**
(43) Date of publication of application: **16.10.2024**
(21) Application number: 22903576.1
(22) Date of filing: 08.12.2022
(51) Int. Cl.: A61F 2/04

(54) **AIRWAY STENT**

(30) Priority: 10.12.2021 CN 202111510936; 10.12.2021 CN 202111510932; 10.12.2021 CN 202111510933
(71) Applicant: Shenzhen Lifetech Respiration Scientific Co., Ltd., Shenzhen, Guangdong 518057 (CN)
(72) Inventor: HU, Longhu, Shenzhen, Guangdong 518057 (CN); LI, Anning, Shenzhen, Guangdong 518057 (CN)
(74) Representative: Prinz & Partner mbB
(86) International application number: PCT/CN2022/137589
(87) International publication number: WO 2023/104157

(57) **Abstract**

The present application relates to the technical field of medical devices. An airway stent includes a stent body and a covering film provided on the stent body; the stent body includes a waist stent segment and a dense mesh bare stent segment provided at an end portion of the waist stent segment; the covering film is provided on the waist stent segment; the mesh density of the dense mesh bare stent segment is higher than the mesh density of the waist stent segment. According to the airway stent in the present application, the stimulation of the airway stent to the inner wall of the trachea can be reduced, thereby effectively avoiding the growth of granulation tissue, and preventing the airway lumen from being narrowed again.

## Description

### Technical Field

The present application relates to the technical field of medical devices, and in particular to an airway stent.

### Background Art

Airway metal stents are an important means to treat trachea and bronchial stenosis, which can quickly reconstruct the airway and relieve dyspnea and other symptoms. Based on the material, airway stents can be divided into metal stents and non-metal stents, and depending on whether there is a covering film, metal stents are divided into covered stents and bare stents.

Early airway stents mainly use bare metal stents, and bare metal stents use nickel-titanium memory alloy mesh stents; bare metal stents have the advantages of being difficult to displace and being able to retain airway secretions. However, since the mesh space of the bare metal stent is virtually large and cannot block the growth of tumor or granulation tissue along the mesh, the malignant tumor easily grows into the lumen of the stent and causes the airway to be narrowed again. Therefore, bare metal stents can only be placed for a short period of time to relieve airway obstruction, and should not be placed for too long.

Based on the above-mentioned problems, the prior art has developed a covered stent, which adds a covering film based on a bare stent, to prevent tumor growth into the stent through the covering film. Due to less stimulation of the inner wall of the airway, the covering film can effectively reduce granulation tissue proliferation. However, the covered stent prevents the mucociliary effect of airway stent, and it is difficult to clean the airway secretions, resulting in the retention of secretions at both ends of the airway, leading to cough, pneumonia, and other complications.

Therefore, there is a need for a new technique that reduces stimulation of the inner wall of the airway by the airway stent while not retaining airway secretions on the surface of the airway stent, thereby reducing the proliferation of granulation tissue.

### Summary of the Application

An object of the present application is at least to solve the problem that the existing covered metal stent easily stimulates the inner wall of the airway, thereby increasing the proliferation of granulation tissue.

The present application proposes an airway stent including a stent body and a covering film provided on the stent body; the stent body includes a waist stent segment and a dense mesh bare stent segment provided at an end portion of the waist stent segment; the covering film is provided on the waist stent segment; the mesh density of the dense mesh bare stent segment is higher than the mesh density of the waist stent segment.

According to the airway stent in the present application, a covering film is provided on the waist stent segment, the stimulation on the inner wall of the airway caused by the waist stent segment is reduced by the covering film, then a dense mesh bare stent segment is provided at the end portion of the waist stent segment, and the pore diameter of the dense mesh bare stent segment is less than that of the waist stent segment; therefore, the airway stent of the present application increases the contact area between the dense mesh bare stent segment and the inner wall of the airway while ensuring the anchoring force between the dense mesh bare stent segment and the inner wall of the airway, reduces the pressure per unit area of the inner wall of the airway, so that the inner wall of the airway is less stimulated, and since the design of the dense mesh bare stent segment is adopted at both ends of the waist stent segment, there is no retention of secretions at both ends of the airway stent. Therefore, by using the airway stent of the present application, it is possible to reduce the stimulation of the airway stent to the inner wall of the airway without causing retention of airway secretions on the surface of the airway stent, thereby reducing the proliferation of granulation tissue and preventing the occurrence of re-stenosis of the airway.

In addition, the airway stent according to the present invention may further have the following additional technical features:
In some embodiments of the present invention, the dense mesh bare stent segment includes a single-layer dense braided mesh structure or a multi-layer braided mesh structure.

In some embodiments of the present invention, the dense mesh bare stent segment includes an inner layer support mesh and an outer layer support mesh; one end of the inner layer support mesh is connected to the waist stent segment, and the other end of the inner layer support mesh is connected to the outer layer support mesh; or, one end of the outer layer support mesh is connected to the waist stent segment, and the other end of the outer layer support mesh is connected to the inner layer support mesh.

In some embodiments of the present invention, the inner layer support mesh is integrally formed with the waist stent segment; an end portion of the inner layer support mesh is turned out and folded back to form the outer layer support mesh, and the inner layer support mesh is connected to the outer layer support mesh by a folded end portion.

In some embodiments of the present invention, the folded end portion is convex relative to the outer layer support mesh in a direction away from the stent body; the outer layer support mesh is provided with at least one convex point support portion, and the convex point support portion has a shape same as or different from that of the folded end portion.

In some embodiments of the present invention, a sandwich film is provided between the inner layer support mesh and the outer layer support mesh.

In some embodiments of the present invention, the covering film includes an outer film provided on an outer surface of the waist stent segment and an inner film provided on an inner surface of the waist stent segment, the inner film and the outer film being combined and fixed through a mesh hole of the waist stent segment.

In some embodiments of the present invention, the stent body is further provided with an extension film covering the inner surface of the dense mesh bare stent segment.

In some embodiments of the present invention, the waist stent segment is provided with a suture line for increasing connection strength between the covering film and the waist stent segment.

In some embodiments of the present invention, a recovery line is provided on the dense mesh bare stent segment at one end of the stent body, the recovery line being threaded along an edge of the dense mesh bare stent segment.

### Brief Description of the Drawings

Fig. 1 is an overall structural diagram of an airway stent in Embodiment 1 of the present application;
Fig. 2 is a structural diagram of a stent body in Embodiment 1 of the present application;
Fig. 3 is a sectional view of a stent body in Embodiment 1 of the present application;
Fig. 4 is a diagram of an airway stent implanted in an airway in Embodiment 1 of the present application;
Fig. 5 is a diagram of growth of granulation on an inner wall of an airway in Embodiment 1 of the present application;
Fig. 6 is a structural diagram of a folded end portion of an airway body in Embodiment 1 of the present application;
Fig. 7 is a sectional view of a folded end portion of an airway body in Embodiment 1 of the present application;
Fig. 8 is a diagram of a second implementation of a folded end portion in Embodiment 1 of the present application;
Fig. 9 is a diagram of a third implementation of a folded end portion in Embodiment 1 of the present application;
Fig. 10 is a structural diagram of a covering film in Embodiment 1 of the present application;
Fig. 11 is a structural diagram of an outer film in Embodiment 1 of the present application;
Fig. 12 is a structural diagram of an outer film and an inner film in Embodiment 1 of the present application;
Fig. 13 is a sectional view of a convex point support portion in Embodiment 2 of the present application;
Fig. 14 is a diagram of another implementation of a convex point support portion in Embodiment 2 of the present application;
Fig. 15 is a structural diagram of an extension film in Embodiment 3 of the present application;
Fig. 16 is a structural diagram of a sandwich film in Embodiment 4 of the present application;
Fig. 17 is a structural diagram of a sandwich film and an extension film in Embodiment 4 of the present application;
Fig. 18 is a structural diagram of a suture line in Embodiment 5 of the present application;
Fig. 19 is a sectional structural diagram of a suture line in Embodiment 5 of the present application;
Fig. 20 is a diagram of a second implementation of a suture line in Embodiment 5 of the present application;
Fig. 21 is a diagram of a third implementation of a suture line in Embodiment 5 of the present application;
Fig. 22 is a diagram of a fourth implementation of a suture line in Embodiment 5 of the present application;
Fig. 23 is a structural diagram of a recovery line in Embodiment 6 of the present application;
Fig. 24 is a sectional structural diagram of a recovery line in Embodiment 6 of the present application;
Fig. 25 is a diagram of another implementation of a recovery line in Embodiment 6 of the present application;
Fig. 26 is an overall structural diagram of an airway stent in Embodiment 7 of the present application;
Fig. 27 is a diagram of an airway stent implanted in an airway in Embodiment 7 of the present application;
Fig. 28 is a structural diagram of a stent body in Embodiment 7 of the present application;
Fig. 29 is a structural diagram of a connecting portion of a stent body in Embodiment 7 of the present application;
Fig. 30 is a structural diagram of another implementation of a connecting portion of a stent body in Embodiment 7 of the present application;
Fig. 31 is a structural diagram of a covering film in Embodiment 7 of the present application;
Fig. 32 is an overall structural diagram of an airway stent in Embodiment 8 of the present application;
Fig. 33 is an overall structural diagram of a stent body in Embodiment 8 of the present application;
Fig. 34 is a structural diagram of a second braided wire in Embodiment 8 of the present application;
Fig. 35 is a structural diagram of another implementation of a second braided wire in Embodiment 8 of the present application;
Fig. 36 is an overall structural diagram of another implementation of an airway stent in Embodiment 8 of the present application;
Fig. 37 is a structural diagram of the connection of a primary braided wire and a secondary braided wire in Embodiment 8 of the present application;
Fig. 38 is a partial structural diagram of a stent body in Embodiment 9 of the present application;
Fig. 39 is a structural diagram of a second braided wire and a third braided wire in Embodiment 9 of the present application; and
Fig. 40 is a structural diagram of another implementation of a second braided wire and a third braided wire in Embodiment 9 of the present application.

Each label in the drawings is represented as follows:
10, stent body; 11, mesh hole; 20, covering film; 21, inner film; 22, outer film; 23, sandwich film; 24, extension film; 25, connecting portion; 30, waist stent segment; 301, second braided wire; 302, third braided wire; 40, dense mesh bare stent segment; 401, first dense mesh segment; 402, second dense mesh segment; 403, first braided wire; 404, primary braided wire; 405, secondary braided wire; 41, inner layer support mesh; 42, outer layer support mesh; 43, folded end portion; 44, convex point support portion; 45, mesh wire concentration point; 50, suture line; 51, suture ring; 60, recovery line; 61, connecting line; 62, operating line; 70, inner wall of airway; 80, tumor; 90, granulation tissue.

### Detailed Description of the Application

Exemplary embodiments of the present application will be described in more detail below concerning the drawings. While the exemplary implementations of the present application are shown in the drawings, the present application can be embodied in various forms and should not be construed as limited to the implementations set forth herein. Rather, these implementations are provided so that the present application will be thorough and complete, and will fully convey the scope of the present application to those skilled in the art.

It is to be understood that the terminology used herein is to describe specific example embodiments only and is not intended to be limiting. As used herein, the singular forms "a", "an", and "the" are intended to include the plural forms as well, unless the context indicates otherwise.

Although the terms first, second, third, etc. may be used herein to describe various elements, components, regions, layers, and/or sections, these elements, components, regions, layers, and/or sections should not be limited by these terms. These terms may only be used to distinguish one element, component, region, layer, or section from another region, layer, or section.

For ease of description, spatially relative terms, such as "inner", "outer", "inside", "outside", "below", "under", "above", "over", and the like, may be used herein to describe one element or feature's relationship to another element or feature as illustrated in the drawings. Such spatially relative terms are intended to encompass different orientations of the device in use or operation in addition to the orientation depicted in the drawings.

For ease of description, the following description uses the terms "proximal" and "distal", where "proximal" refers to the end proximal to the operator and "distal" refers to the end distal to the operator, and the phrase "axial direction", which in this patent should be understood to mean the direction in which the interventional instrument is advanced and advanced, with the direction perpendicular to the "axial direction" being defined as the "radial direction".

### Embodiment 1

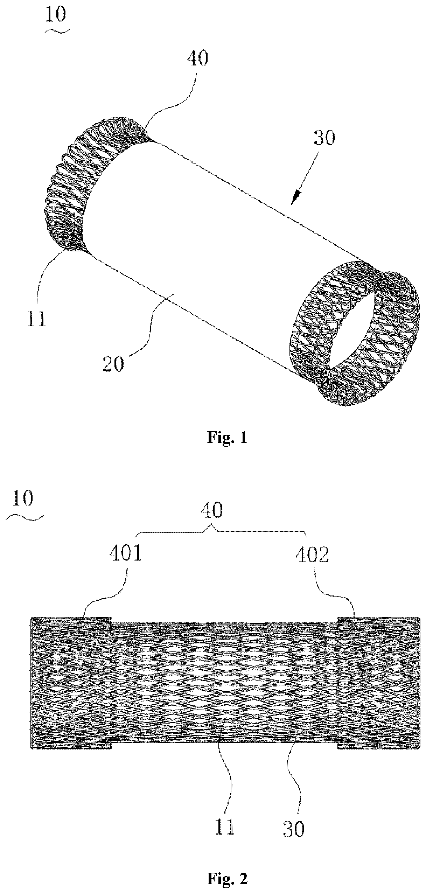

An airway stent, as shown in Figs. 1 and 2, includes a stent body 10 and a covering film 20 provided on the stent body 10. The stent body 10 includes a waist stent segment 30 and a dense mesh bare stent segment 40 provided at one end of the waist stent segment 30. The stent body 10 is formed by braiding braided wires, with gaps between adjacent braided wires enclose to form mesh holes 11; the density of the mesh holes 11 of the dense mesh bare stent segment 40 is greater than the density of the mesh holes 11 of the waist stent segment 30, and the covering film 20 is provided on the waist stent segment 30. First, in the present application, a covering film 20 is provided on the waist stent segment 30, so that the covering film 20 can reduce the stimulation on the inner wall of the airway caused by the waist stent segment 30 and can prevent the tumor from growing toward the airway stent.

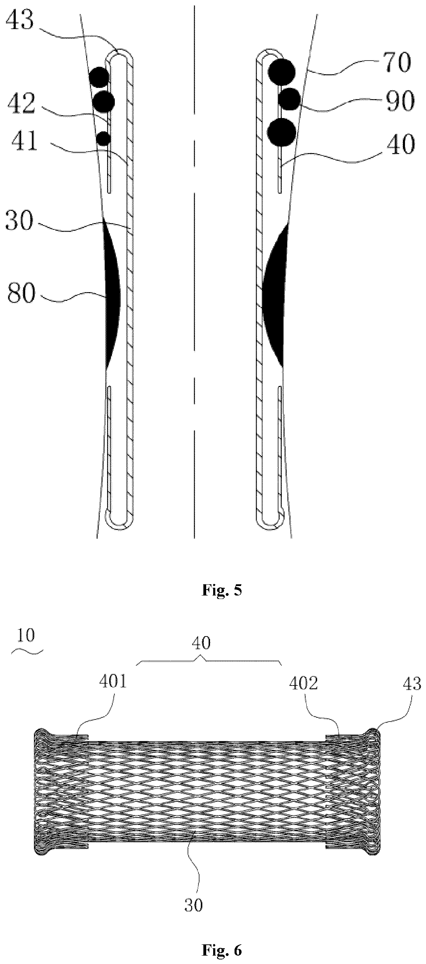

In the embodiment, as shown in conjunction with Fig. 5, the dense mesh bare stent segment 40 is a stent segment provided with an outer non-film structure, and the density of the mesh holes 11 is the number of the mesh holes 11 per unit area. That is, the waist stent segment 30 of the present application is provided with a covering film 20, and at least one end of the waist stent segment 30 is provided with a dense mesh bare stent segment 40, to reduce the stimulation of the stent body 10 on the inner wall of the airway 70. Since the outer side of the dense mesh bare stent segment 40 is provided without a film structure, the ciliary function of the inner wall of airway 70 is not affected, ensuring that airway secretions are not retained on the airway body 10. Since the airway body 10 is provided with a dense mesh bare stent segment 40 which is provided without a film structure, and the bare stent easily causes stimulation to the airway inner wall 70, the density of the mesh holes 11 of the dense mesh bare stent segment 40 is set to be greater, so that the contact area between the dense mesh bare stent segment 40 and the airway inner wall 70 can be increased, and the pressure of the dense mesh bare stent segment 40 on the airway inner wall 70 as a whole can be reduced, thereby effectively reducing the stimulation of the end portion of the stent body 10 on the airway inner wall 70 and reducing the growth of granulation tissue 90.

In the present application, at least one end of the waist stent segment 30 is provided with a dense mesh bare stent segment 40, or both ends of the waist stent segment 30 are provided with dense mesh bare stent segments 40. Specifically, the two ends of the waist stent segment 30 are provided with a dense mesh bare stent segment 40. Through the above-mentioned technical solution, the two ends of the airway body 10 are exposed with the dense mesh bare stent segment 40 to ensure an anchoring force between the stent body 10 and the inner wall of the airway. Since the two ends of the waist stent segment 30 are designed with the dense mesh bare stent segment 40, there is a gap between the dense mesh bare stent segment 40, and there is no secretion retention at the two ends of the stent body 10.

Therefore, by using the airway stent of the present application, not only can the anchoring force be ensured, but also the stimulation of the inner wall of the airway by the stent body 10 can be reduced without retaining airway secretions on the surface of the airway stent, thereby reducing the proliferation of granulation tissue, and effectively avoiding the occurrence of airway restenosis.

The dense mesh bare stent segment 40 in the present application can adopt a multilayer braided mesh structure arranged in a staggered manner, and can also adopt a single-layer braided mesh that is densely braided; when a single-layer braided mesh densely braided mesh is adopted, one braided wire can be used for the densely braided mesh, and two or more different braided wires can also be used for hybrid braids.

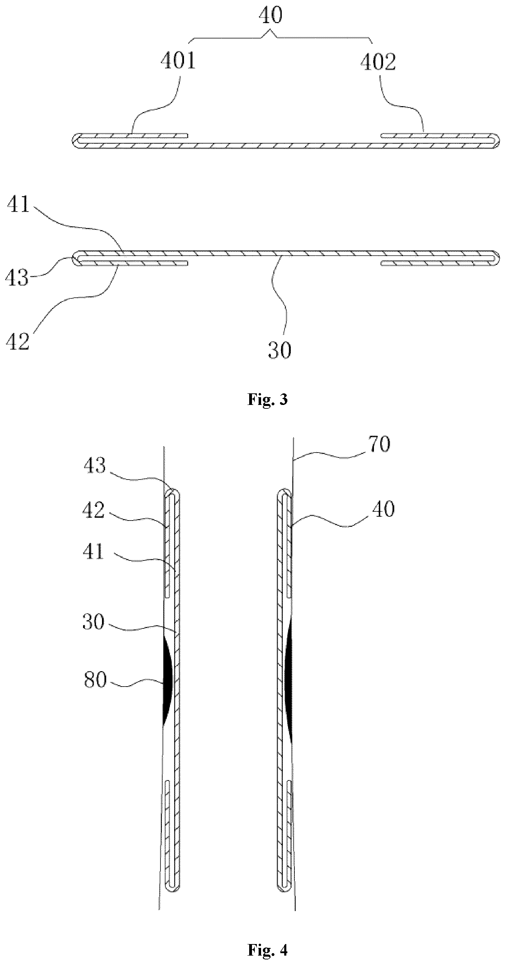

In the embodiment, as shown in Fig. 3, the dense mesh bare stent segment 40 adopts a double-layer braided mesh structure. The double-layer braided mesh structure of the dense mesh bare stent segment 40 includes an inner layer support mesh 41, one end of inner layer support mesh 41 is connected to the waist stent segment 30, and an outer layer support mesh 42, the other end of inner layer support mesh 41 is connected to the outer layer support mesh 42. In other embodiments, one end of the outer layer support mesh 42 is connected to the waist stent segment 30 and the other end of the outer layer support mesh 42 is connected to the inner layer support mesh 41.

In this embodiment, one end of the inner layer support mesh 41 is connected to the waist stent segment 30, the other end of the inner layer support mesh 41 is connected to the outer layer support mesh 42, and the inner layer support mesh 41 and the outer layer support mesh 42 are connected by a folded end portion 43.

As shown in Figs. 4 and 5, both end portions of the existing airway stent are easily granulated as the implantation time increases. It has been shown in the literature that the granulation tissue 90 at both ends of the stent body 10 is primarily due to sharp material stimulation at both ends of the stent body 10. However, the end of the dense mesh bare stent segment 40 formed by everting and folding back in the present application does not have a sharp part of a conventional bare support, namely, the inner layer support mesh 41, and the outer layer support mesh 42 are connected via a folded end portion 43.

On the one hand, since the folded end portion 43 is formed by the tuck and thus has a smooth surface, and the smooth surface stimulates the airway inner wall 70 much less than the sharp end portion of the conventional bare stent, both ends of the stent body 10 are less likely to cause the granulation tissue 90 to grow due to the stimulation.

On the other hand, since the two ends of the stent body 10 are in a turned out and doubled structure, thereby forming a densely braided end portion, the contact area between the two end portions of the stent body 10 and the airway inner wall 70 increases, reducing the pressure received per unit area of the airway inner wall 70, making the airway inner wall 70 less stimulating, and further reducing the proliferation of granulation tissue 90.

In addition, the airway stent of the embodiment can slow down the time for granulation to grow into the inside of the stent even if granulation is generated at both ends of the stent body 10. As the granulation tissue 90 proliferates, it first contacts the outer layer support mesh 42 on the outer side, and if the granulation tissue 90 grows beyond the outer layer support mesh 42, it again contacts the inner layer support mesh 41 on the inner side, the inner layer support mesh 41 acts as a second barrier, again blocking the granulation tissue 90 from growing inside the stent. The outer layer support mesh 42 and the inner layer support mesh 41 act as a double-layer barrier to increase the time for granulation tissue 90 to grow into the lumen of the airway stent and to allow more time for the physician and patient to resolve.

Specifically, the inner layer support mesh 41 is integrally formed with the waist stent segment 30, and the end portion of the inner layer support mesh 41 is turned out and folded back to form the outer layer support mesh 42.

In other embodiments, when one end of the outer layer support mesh 42 is connected to the waist stent segment 30 and the other end of the outer layer support mesh 42 is connected to the inner layer support mesh 41, the end portion of the outer layer support mesh 42 may be inverted and folded back to form the inner layer support mesh 41. That is, regardless of whether the dense mesh bare stent segment 40 is formed by eversion or inversion, it suffices that a double-layer braided mesh structure be formed so that the density of the mesh holes 11 of the dense mesh bare stent segment 40 is greater than the density of the meshes of the waist stent segment 30.

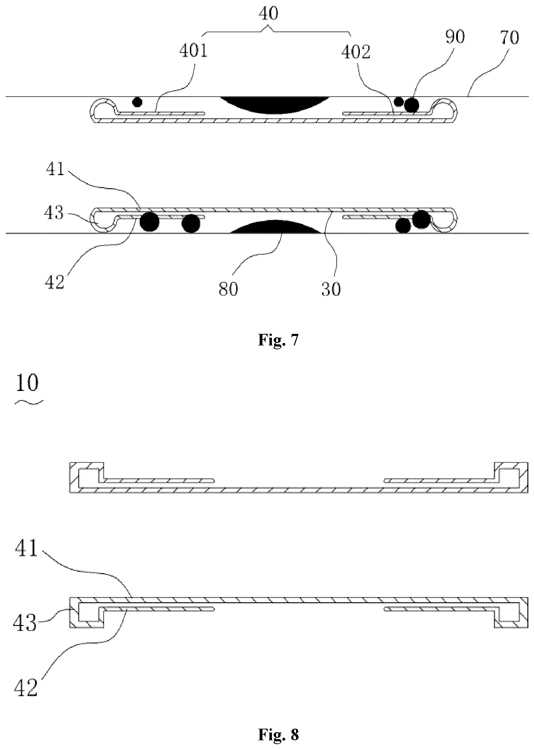

In this embodiment, as shown in Figs. 6 and 7, the two dense mesh bare stent segments 40 respectively arranged at the two ends of the waist stent segment 30 include a first dense mesh segment 401 arranged at one end of the waist stent segment 30 and a second dense mesh segment 402 arranged at the other end of the waist stent segment 30, and after the stent body 10 is braided into a mesh tube structure by one or more braided wires, the two ends of the mesh tube structure are turned out and then folded into a first dense mesh segment 401 and a second dense mesh segment 402 respectively. The structure of the first dense mesh segment 401 and the structure of the second dense mesh segment 402 can be the same or different, and the physician can make a selection according to the actual situation of the patient. For example, the length of the first dense mesh segment 401 and the second dense mesh segment 402 are different, or the structure of the folded end portion 43 of the first dense mesh segment 401 and the second dense mesh segment 402 are different, etc.

The folded end portion 43 is convex in a direction away from the stent body 10 with respect to the outer layer support mesh 42, that is, the outer diameter of the folded end portion 43 at both ends of the stent body 10 is greater than the outer diameter of the waist stent segment 30.

The structure of the folded end portion 43 is a circular arc-shaped structure, such as a circular or spherical structure. That is, an arc-shaped transition is adopted at the folded portion of the inner layer support mesh 41 and the outer layer support mesh 42, so that the contact surface between the folded end portion 43 and the inner wall of the airway 70 smoothly transitions, and the stimulation of the inner wall of the airway 70 by the stent body 10 is reduced.

After the stent body 10 is implanted, the arc-shaped surface of the folded end portion 43 is in contact with the inner wall of the airway 70 for keeping the stent body 10 fixed, and at the same time, the cleaning effect of the airway mucociliary at the two end portions of the stent body 10 is ensured, the retention of airway secretions is reduced, and the proliferation of granulation tissue 90 can be organized.

The main function of the airway stent is that when the stent is implanted at the site where the malignant airway lesion causes airway stenosis, the airway body 10 compresses the tumor 80 to restore central airway ventilation. At this time, the main mechanism of fixing the airway body 10 in the airway is that the radial expansion force of the waist stent segment 30 located at the middle of the stent body 10 interacts with the tumor 80 without the stent body 10 being displaced.

Generally, for a malignant tumor 80, radiotherapy is usually performed after stent implantation. After radiotherapy, the tumor 80 will gradually shrink, and as the tumor 80 shrinks, the force between waist stent segment 30 and the tumor 80 will relatively decrease. When this occurs, prior art airway stents are prone to displacement.

However, in the present application, since the folded end portion 43 is convex with respect to the outer layer support mesh 42 in a direction facing away from the stent body 10, the outer diameter of the folded end portion 43 is greater than the outer diameter of the waist stent segment 30. After the tumor 80 shrinks, although the acting force between the waist stent segment 30 and the tumor 80 decreases, the acting force between the folded end portion 43 and the inner wall 7 of the airway relatively increases, thereby maintaining the overall acting force between the stent body 10 and the airway, and therefore the airway stent of the present application is not easily displaced.

In other embodiments, shown in connection with Fig. 8, the folded end portion 43 may also be square-shaped. The above arrangement is advantageous in that it is possible to increase the contact area between the folded end portion 43 and the inner wall of the airway 70 and increase the anchoring force of the stent body 10 to the inner wall of the airway 70.

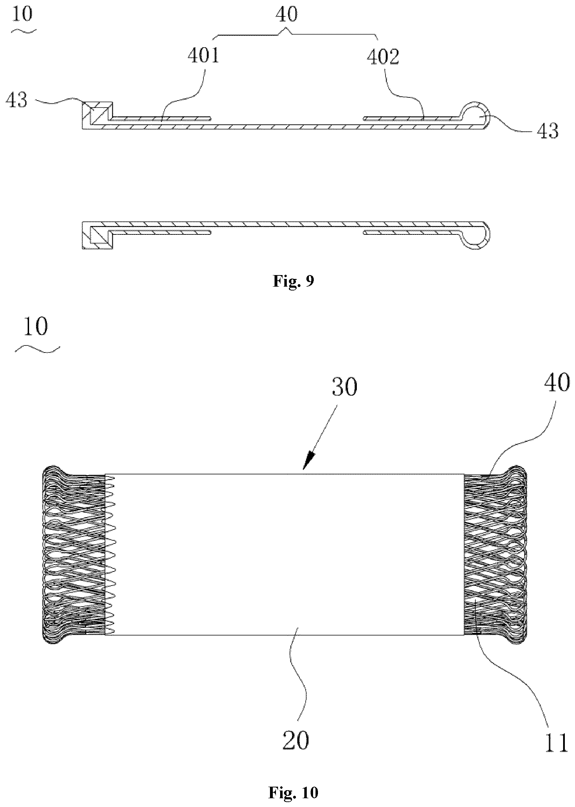

In other embodiments, as shown in Fig. 9, when the configuration of the folded end portion 43 of the first dense mesh segment 401 and the second dense mesh segment 402 is different, the folded end portion 43 of the first dense mesh segment 401 is square, and the folded end portion 43 of the second dense mesh segment 402 is arc-shaped. The advantage of the above arrangement is that the physician can select different shapes of the folded end portion 43 to accommodate patients with different conditions, depending on the actual condition of the patient.

Further, as shown in conjunction with Figs. 10-12, the covering film 20 includes an outer film 22 provided on the outer surface of the waist stent segment 30. To further prevent the malignant tumor 80 in the airway from growing into the stent body 10, resulting in restenosis of the inner wall of the airway 70 or difficulty in removal of the stent body 10, this embodiment is provided with a biocompatible polymer covering film 20 on the outer surface of the waist stent segment 30 for further preventing the malignant tumor 80 from growing into the stent body 10.

The material of the covering film 20 can be selected from PET, PTFE, ePTFE, silicone, polyurethane, etc. and the thickness of the covering film 20 is adjusted according to the size of the braided wire of the stent body 10 and the inner and outer diameters of the stent body 10, which is not limited in the present application.

To enhance the bonding strength between the covering film 20 and the stent body 10 and further prevent tumor ingrowth, the covering film 20 of the present application further includes an inner film 21 provided on the inner surface of the waist stent segment 30, and since the stent body 10 has the mesh hole 11, the inner film 21 and the outer film 22 can be bonded and fixed through the mesh hole 11 of the waist stent segment 30. The inner film 21 and the outer film 22 can be fixed by hot melting or by bonding, and the materials of the inner film 21 and the outer film 22 can be selected to be the same or different.

Therefore, the embodiment provides the dense mesh bare stent segment 40 at both ends of the waist stent segment 30, and the density of the mesh hole 11 of the dense mesh bare stent segment 40 is greater than the density of the mesh hole 11 of the waist stent segment 30. At the same time, the airway stent of the present application increases the contact area between the dense mesh bare stent segment 40 and the airway inner wall 70 while ensuring the anchoring force between the dense mesh bare stent segment 40 and the airway inner wall 70, and reduces the pressure per unit area of the airway inner wall 70, so that the stimulation of the airway inner wall 70 becomes smaller, and since the design of the dense mesh bare stent segment 40 is adopted at both ends of the waist stent segment 30, there is no retention of secretions at both ends of the stent body 10.

In conclusion, by using the airway stent of the present application, it is possible to reduce the stimulation of the inner wall of the airway by the airway stent while not retaining airway secretions on the surface of the airway stent, thereby reducing the proliferation of granulation tissue, and avoiding the occurrence of re-stenosis of the airway.

### Embodiment 2

Embodiment 2 of the present application provides an airway stent, and as shown in Figs. 1-3, the stent body 10 includes a waist stent segment 30 and two dense mesh bare stent segments 40 respectively arranged at two ends of the waist stent segment 30, the density of the mesh hole 11 of the dense mesh bare stent segment 40 is greater than the density of the mesh hole 11 of the waist stent segment 30, and a covering film 20 is arranged on the waist stent segment 30. The dense mesh bare stent segment 40 is a double-layer braided mesh structure, and the dense mesh bare stent segment 40 of the double-layer braided mesh structure includes an inner layer support mesh 41 and an outer layer support mesh 42. One end of the inner layer support mesh 41 is connected to the waist stent segment 30, and the inner layer support mesh 41 is integrally formed with the waist stent segment 30, and the other end of the inner layer support mesh 41 is connected to the outer layer support mesh 42. In conjunction with Fig. 6, an end portion of the inner layer support mesh 41 is turned out and folded back to form an outer layer support mesh 42, and the inner layer support mesh 41 and the outer layer support mesh 42 are connected via a folded end portion 43, and the folded end portion 43 is convex with respect to the outer layer support mesh 42 in a direction away from the stent body 10.

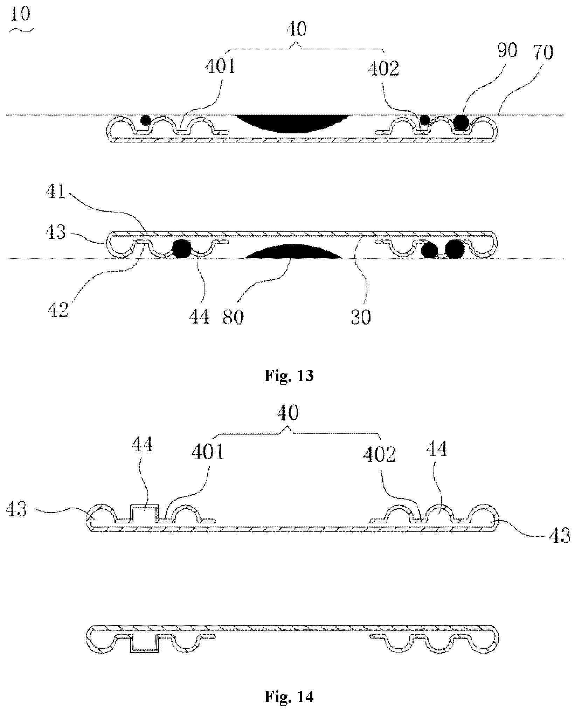

As shown in Figs. 13 and 14, in the second embodiment, the outer layer support mesh 42 is provided with at least one convex point support portion 44, and the convex point support portion 44 is the same or different in shape from the folded end portion 43. The convex point support portion 44 is integrally formed with the outer layer support mesh 42, and the convex point support portion 44 is formed by the outer layer support mesh 42 protruding in a direction away from the stent body 10.

The convex point support portions 44 may be one or more, and when the convex point support portions 44 are plural, the shapes of the plural convex point support portions 44 may be the same or different. The shape of the convex point support portion 44 is preferably a circular arc shape and may be provided in another shape such as a square shape according to actual needs.

The embodiment can strengthen the anchoring force between the stent body 10 and the inner wall of the airway 70 by adding the convex point support portions 44 on the outer layer support mesh 42 and can reduce the pressure between the folded end portion 43 and each convex point support portion 44 and the inner wall of the airway 70, to reduce the stimulation of the inner wall of the airway 70 by the stent body 10 and avoid the proliferation of granulation tissue 90.

In other embodiments, two dense mesh bare stent segments 40 provided at each end of the waist stent segment 30 include a first dense mesh segment 401 provided at one end of the waist stent segment 30 and a second dense mesh segment 402 provided at the other end of the waist stent segment 30.

When the structure of the convex point support portions 44 of the first dense mesh segment 401 and the second dense mesh segment 402 is different, the convex point support portions 44 of the first dense mesh segment 401 may both be circular arc-shaped or one of the convex point support portions 44 may be circular arc-shaped, and the convex point support portions 44 of the second dense mesh segment 402 may both be square-shaped or one of the convex point support portions 44 may be square-shaped. The advantage of the above arrangement is that the physician can select different shapes of the convex point support portion 44 to accommodate patients with different conditions, depending on the actual condition of the patient.

To sum up, the embodiment increases the anchoring force between the stent body 10 and the inner wall of the airway 70 by adding the convex point support portion 44, reduces the overall pressure between the stent body 10 and the inner wall of the airway 70, reduces the stimulation of the inner wall of the airway 70 by the stent body 10, and effectively avoids the proliferation of granulation tissue 90.

### Embodiment 3

Embodiment 3 of the present application provides an airway stent, and as shown in Figs. 1-3, the stent body 10 includes a waist stent segment 30 and two dense mesh bare stent segments 40 respectively arranged at two ends of the waist stent segment 30, the density of the mesh holes 11 of the dense mesh bare stent segment 40 is greater than the density of the mesh holes 11 of the waist stent segment 30, and a covering film 20 is arranged on the waist stent segment 30. As shown in Fig. 5, the dense mesh bare stent segment 40 is a double-layer braided mesh structure, and the dense mesh bare stent segment 40 of the double-layer braided mesh structure includes an inner layer support mesh 41 and an outer layer support mesh 42. One end of the inner layer support mesh 41 is connected to the waist stent segment 30 and the other end of the inner layer support mesh 41 is connected to the outer layer support mesh 42.

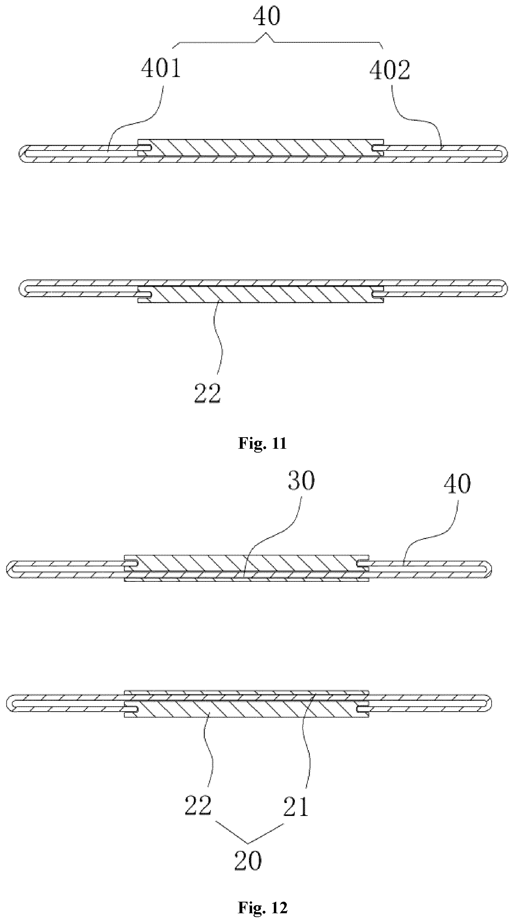

As shown in Fig. 12, the covering film 20 includes an outer film 22 provided on the outer surface of the waist stent segment 30 and an inner film 21 provided on the inner surface of the waist stent segment 30, and the inner film 21 and the outer film 22 are fixed by bonding, such as heat fusion bonding, through the mesh hole 11 of the waist stent segment 30.

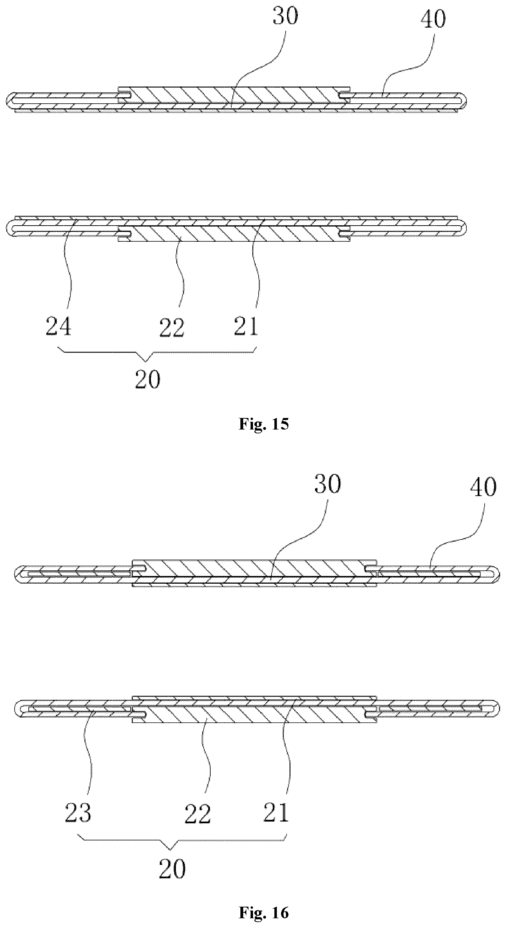

As shown in Fig. 15, in the embodiment, to further prevent the tumor 80 from ingrowth and prevent the proliferation of granulation tissue 90 at both ends of the stent body 10, an extension film 24 covering the inner surface of the dense mesh bare stent segment 40 is further provided in the stent body 10, and the extension film 24 is integrally provided with the inner film 21 to cover the total internal surface of the stent body 10.

The material of the extension film 24 can be selected from PET, PTFE, ePTFE, silicone, polyurethane, etc. and the thickness of the extension film 24 is set according to practical requirements. When the extension film 24 is integrally provided with the inner film 21, the material of the extension film 24 is the same as that of the inner film 21.

In other embodiments, the extension film 24 and the inner film 21 may be provided separately from the inner film 21, and the materials of the extension film 24 and the inner film 21 may be the same or different.

Since the extension film 24 is also added to the inner surface of the dense mesh bare stent segment 40, the covering film 20 of the embodiment can cover the total internal surface of the stent body 10, preventing not only tumor 80 ingrowth but also granulation tissue 90 ingrowth at both ends of the stent body 10. At the same time, the turned-out part of the stent body 10, i.e. the outer layer support mesh 42, maintains the bare stent state, and the outer surface of the outer layer support mesh 42 is not covered with the covering film 20, so that the cleaning capacity of the mucociliary in the airway remains unchanged, and the retention of secretions on the inner wall of the airway 70 can be effectively removed.

In other embodiments, the outer film 22 may also partially cover the dense mesh bare stent segment 40 to increase the attachment force between the dense mesh bare stent segment 40 and the waist stent segment 30.

In conclusion, this embodiment prevents the proliferation of granulation tissue 90 at both ends of the stent body 10 while preventing the tumor 80 from ingrowth by providing the extension film 24 covering the inner surface of the dense mesh bare stent segment 40 on the inner side of the stent body 10.

### Embodiment 4

Embodiment 4 of the present application provides an airway stent, and as shown in Figs. 1-3, the stent body 10 includes a waist stent segment 30 and two dense mesh bare stent segments 40 respectively arranged at two ends of the waist stent segment 30, the density of the mesh hole 11 of the dense mesh bare stent segment 40 is greater than the density of the mesh hole 11 of the waist stent segment 30, and a covering film 20 is arranged on the waist stent segment 30. As shown in Fig. 5, the dense mesh bare stent segment 40 is a double-layer braided mesh structure, and the dense mesh bare stent segment 40 of the double-layer braided mesh structure includes an inner layer support mesh 41 and an outer layer support mesh 42. One end of the inner layer support mesh 41 is connected to the waist stent segment 30 and the other end of the inner layer support mesh 41 is connected to the outer layer support mesh 42.

As shown in Fig. 12, the covering film 20 includes an outer film 22 provided on the outer surface of the waist stent segment 30 and an inner film 21 provided on the inner surface of the waist stent segment 30, and the inner film 21 and the outer film 22 are fixed by bonding, such as heat fusion bonding, through the mesh hole 11 of the waist stent segment 30.

As shown in Fig. 16, in this embodiment, to further prevent tumor 80 from ingrowth and the proliferation of granulation tissue 90, a sandwich film 23 is provided between the inner layer support mesh 41 and the outer layer support mesh 42. By the presence of the sandwich film 23, it is possible not only to prevent tumor 80 ingrowth and proliferation of granulation tissue 90, but also to maintain the mucociliary cleaning capacity in the inner wall of the airway 70.

Specifically, the inner side surface of the stent body 10 is covered with an inner film 21, the outer side surface of the stent body 10 is covered with an outer film 22, the interlayer portion between the outer layer support mesh 42 and the inner layer support mesh 41, which are folded back at both ends of the stent body 10, is provided with a sandwich film 23, and the sandwich film 23 can be closely adhered to the inner layer support mesh 41.

The material of the sandwich film 23 can be selected from PET, PTFE, ePTFE, silica gel, polyurethane, etc. and the thickness of the sandwich film 23 is set according to actual needs.

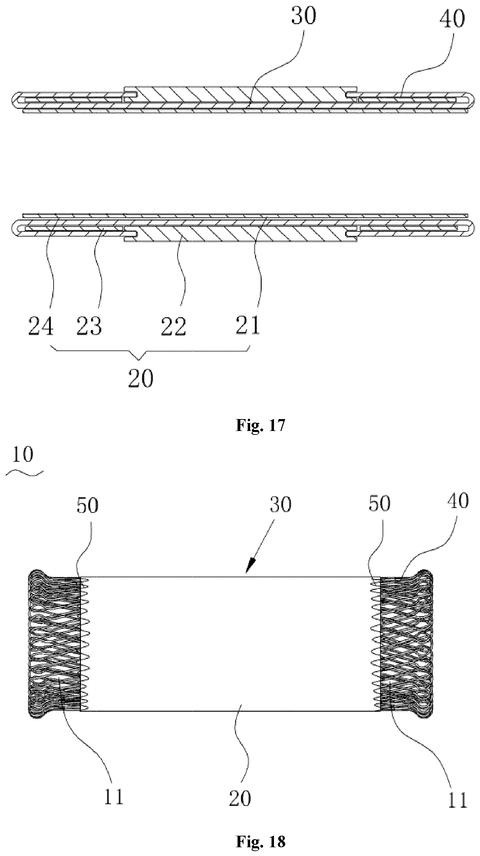

In other embodiments, as shown in Fig. 17, to improve the weldability of the sandwich film 23, the stent body 10 is further provided with an extension film 24 covering the inner surface of the dense mesh bare stent segment 40, the extension film 24 is integrally provided with the inner film 21 to cover the total internal surface of the stent body 10. The sandwich film 23 and the extension film 24 are combined and fixed via the mesh hole 11 of the inner layer support mesh 41; the sandwich film 23 and the extension film 24 can be fixed by hot-melting or by adhesion.

To sum up, this embodiment prevents tumor 80 ingrowth and proliferation of granulation tissue 90 without affecting the mucociliary cleaning capacity of the airway inner wall 70 by adding a sandwich film 23 between inner layer support mesh 41 and outer layer support mesh 42.

### Embodiment 5

Embodiment 5 of the present application provides an airway stent, and as shown in Figs. 1-3, the stent body 10 includes a waist stent segment 30 and two dense mesh bare stent segments 40 respectively arranged at two ends of the waist stent segment 30, the density of the mesh holes 11 of the dense mesh bare stent segment 40 is greater than the density of the mesh holes 11 of the waist stent segment 30, and a covering film 20 is arranged on the waist stent segment 30. The dense mesh bare stent segment 40 is a double-layer braided mesh structure, and the dense mesh bare stent segment 40 of the double-layer braided mesh structure includes an inner layer support mesh 41 and an outer layer support mesh 42. One end of the inner layer support mesh 41 is connected to the waist stent segment 30 and the other end of the inner layer support mesh 41 is connected to the outer layer support mesh 42.

As shown in Fig. 12, the covering film 20 includes an outer film 22 provided on the outer surface of the waist stent segment 30 and an inner film 21 provided on the inner surface of the waist stent segment 30, and the inner film 21 and the outer film 22 are fixed by bonding, such as heat fusion bonding, through the mesh hole 11 of the waist stent segment 30.

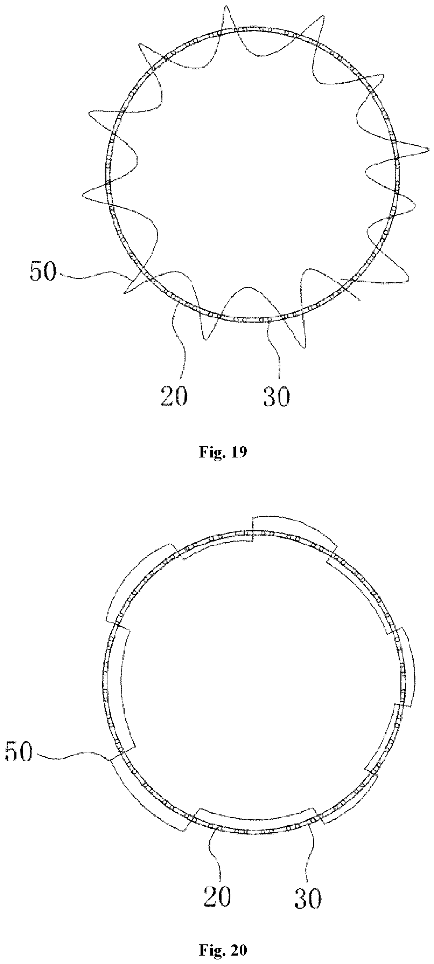

As shown in Fig. 18 and Fig. 19, in the embodiment, to further improve the bonding strength of the covering film 20 to the stent body 10 and prevent the covering film 20 from being separated from the stent, a suture line 50 for increasing the bonding strength of the covering film 20 to the waist stent segment 30 is provided on the waist stent segment 30.

In other embodiments, the covering film 20 may also partially overlie the dense mesh bare stent segment 40 and the suture line 50 sewn to the dense mesh bare stent segment 40.

In this embodiment, the suture line 50 is passed through the mesh hole 11 of the waist stent segment 30 or the dense mesh bare stent segment 40 and passes inside and outside around the outer edge of the stent body 10 and the edge of the covering film 20 to form an undulating linear structure, and then is wound and tightened back and forth so that the covering film 20 is closely adhered to the braided wire of the stent body 10. In this embodiment, the suture line 50 is formed around the outer edge of the stent body 10 at least once, thereby completely sewing and fixing one complete turn of the covering film 20 to the stent body 10.

The suture line 50 may be passed through every other mesh hole 11 or may be passed through every multiple mesh hole 11, and as shown in Fig. 20, the suture line 50 may be passed through every four mesh holes 11. When the suture line 50 passes through the covering film 20, a puncture hole is generated, and if the puncture hole is too large, the strength of the covering film 20 may be affected. Therefore, the interval at which the suture line 50 passes may be selected depending on the material of the suture line 50 and the like, and the embodiment is not limited thereto.

Since too many puncture holes may affect the strength of the covering film 20, the suture line 50 shares one puncture hole when passing through the same mesh hole 11 from inside to outside and from outside to inside, thereby reducing the number of puncture holes.

The suture line 50 is preferably made of PET, PTFE, ePTFE, etc. and the outer diameter of the suture line 50 is determined according to the wire diameter of the braided wire of the stent body 10 and the thickness of the covering film 20, which is not limited in the present application.

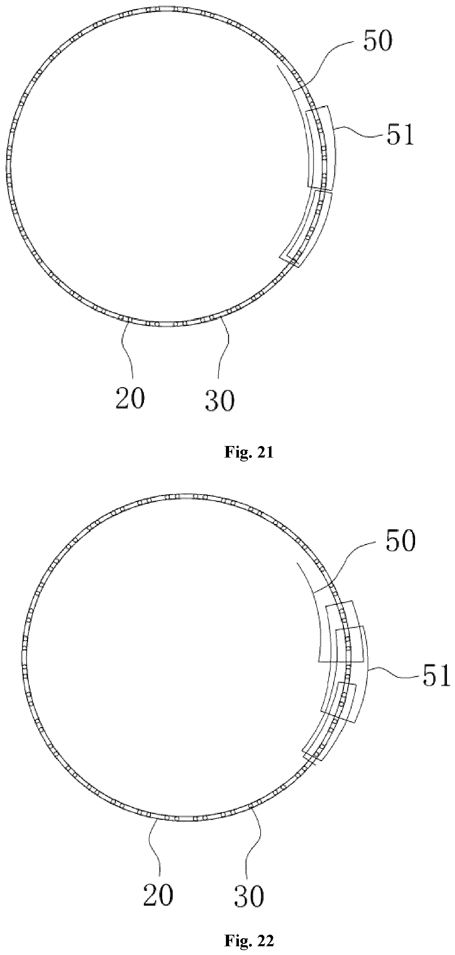

In other embodiments, as shown in Figs. 21 and 22, the covering film 20 may also be partially sutured, i.e. the suture lines 50 are provided less than an entire circumference around the outer edge of the stent body 10. Through partial suturing, the structural strength between the covering film 20 and the stent body 10 can be enhanced while ensuring the structural strength of the covering film 20.

When the covering film 20 is partially sewn, the suture line 50 may be sewn in a laminate manner. Specifically, the suture line 50 is arranged at the edge of the covering film 20, and the covering film 20 is tensioned through the suture line 50 through internal and external puncture, and the suture line 50 is misaligned in the puncture hole during internal and external puncture.

With the above-described embodiment, the suture line 50 is formed into several suture rings 51 connected end-to-end or overlapped in a staggered manner, each of the suture rings 51 passes through the mesh hole 11 of the stent body 10. Preferably, adjacent suture rings 51 have overlapping portions. Since the adjacent suture rings 51 have overlapping portions, the sewing strength of the suture line 50 as a whole is increased, achieving the object of enhancing the sewing strength without affecting the strength of the covering film 20.

To sum up, this embodiment enhances the bonding strength of the covering film 20 to the stent body 10 by adding suture line 50 to the stent body 10 and sewing the covering film 20 to the waist stent segment 30 through the suture line 50, thereby effectively preventing the covering film 20 from being detached from the stent body 10.

### Embodiment 6

Embodiment 6 of the present application provides an airway stent, and as shown in Figs. 1 and 2, the stent body 10 includes a waist stent segment 30 and two dense mesh bare stent segments 40 respectively arranged at two ends of the waist stent segment 30, the density of the mesh holes 11 of the dense mesh bare stent segment 40 is greater than the density of the mesh holes 11 of the waist stent segment 30, and a covering film 20 is arranged on the waist stent segment 30.

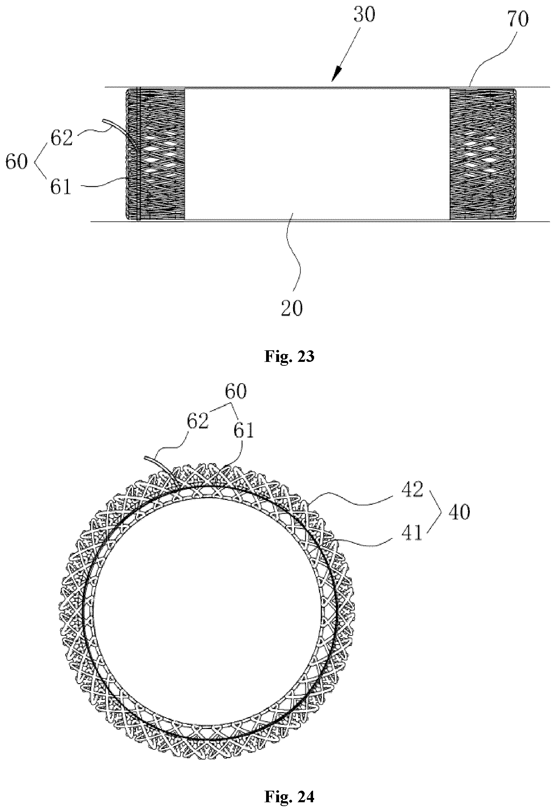

As shown in Fig. 23, in this embodiment, to further facilitate withdrawal or recovery of the stent after the stent body 10 is implanted in the airway, a recovery line 60 is provided on the stent body 10. A recovery line 60 is provided on the dense mesh bare stent segment 40 at one end of the stent body 10, and the recovery line 60 passes along the edge of the dense mesh bare stent segment 40.

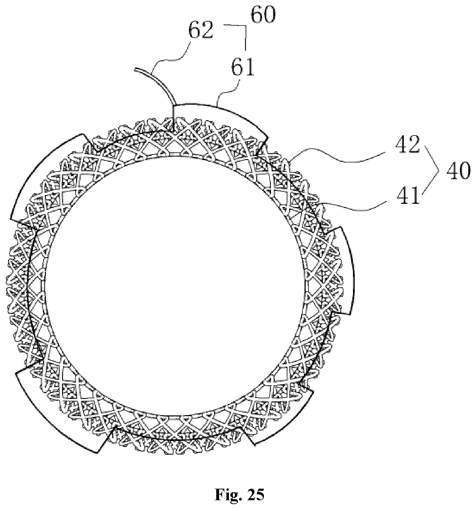

As shown in Figs. 24 and 25, the dense mesh bare stent segment 40 includes an inner layer support mesh 41 and an outer layer support mesh 42, and the recovery line 60 is at least partially arranged between the inner layer support mesh 41 and the outer layer support mesh 42. The recovery line 60 includes a connecting line 61 and an operating line 62; the connecting line 61 is at least partially provided between the inner layer support mesh 41 and the outer layer support mesh 42; one end of the operating line 62 is connected to the connecting line 61, and the other end of the operating line 62 is located outside the stent body 10 for a physician to operate.

In the specific operation, the physician pulls the operating line 62, and the connecting line 61 is driven by the operating line 62 to reduce the diameter of the opening of the dense mesh bare stent segment 40. Since the diameter of the opening of the dense mesh bare stent segment 40 is reduced, the opening of the dense mesh bare stent segment 40 is separated from the inner wall of the airway 70, so that the force between the stent body 10 and the inner wall of the airway 70 is reduced, facilitating the removal of the airway stent by the physician.

When the dense mesh bare stent segment 40 is a single-layer dense braided mesh structure, the connecting lines 61 run along the edges of the dense mesh bare stent segment 40. When the dense mesh bare stent segment 40 is a two-layer or multi-layer braided mesh structure, the connecting line 61 may be partially or fully located between the two layers of braided mesh.

In this embodiment, the dense mesh bare stent segment 40 is a double-layer braided mesh structure. The connecting line 61 may be located partly between the inner layer support mesh 41 and the outer layer support mesh 42 and partly outside the outer layer support mesh 42. The connecting line 61 may also be located entirely between the inner layer support mesh 41 and the outer layer support mesh 42, and when the connecting line 61 is located entirely between the inner layer support mesh 41 and the outer layer support mesh 42, the connecting line 61 is subjected to less friction when moving, and less force is required to shrink the opening of dense mesh bare stent segment 40 of the dense mesh, to facilitate the recovery operation of the doctor.

In summary, the airway stent provided in the present application is provided with a covering film on the waist stent segment, the stimulation on the inner wall of the airway caused by the waist stent segment is reduced by the covering film, and then dense mesh bare stent segments are provided at both ends of the waist stent segment, and the pore diameter of the dense mesh bare stent segment is less than that of the waist stent segment. Therefore, the airway stent of the present application increases the contact area between the dense mesh bare stent segment and the inner wall of the airway while ensuring the anchoring force between the dense mesh bare stent segment and the inner wall of the airway reduces the pressure per unit area of the inner wall of the airway so that the inner wall of the airway is less stimulated, and since the design of the dense mesh bare stent segment is used at the two ends of the waist stent segment, there is no secretion retention at the two ends of the airway stent. Therefore, by using the airway stent of the present application, it is possible to reduce the stimulation of the inner wall of the airway by the airway stent while not retaining airway secretions on the surface of the airway stent, thereby reducing the proliferation of granulation tissue, and avoiding the occurrence of airway restenosis.

### Embodiment 7

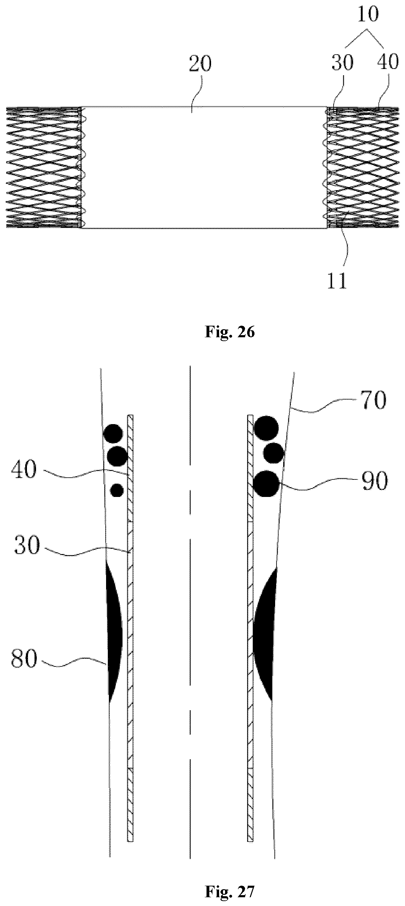

Embodiment 7 of the present application provides an airway stent, as shown in Figs. 26-28, including a stent body 10 and a covering film 20 arranged on the stent body 10; the stent body 10 includes a waist stent segment 30 and a dense mesh bare stent segment 40 arranged at the end portion of the waist stent segment 30, and the covering film 20 is arranged on the waist stent segment 30. In the embodiment, the wire diameter of the mesh of the dense mesh bare stent segment 40 is less than the wire diameter of the mesh of the waist stent segment 30, and the dense mesh bare stent segment 40 has a single-layer dense braided mesh structure.

A mesh hole 11 is formed between the mesh wires of the stent body 10. The dense mesh bare stent segment 40 is a stent segment provided without a film structure on the outside, and at least one end of the waist stent segment 30 is provided with the dense mesh bare stent segment 40. Since the dense mesh bare stent segment 40 is provided without a film structure on the outside, the ciliary function of the inner wall of the airway 70 is not affected, ensuring that airway secretions do not remain on the airway body. In addition, the waist stent segment 30 of the present application is provided with a covering film 20, to reduce the stimulation of the stent body 10 on the inner wall of the airway 70.

Specifically, the stent body 10 includes a plurality of first braided wires 403 and a plurality of second braided wires 301; the mesh wires of the dense mesh bare stent segment 40 are first braided wires 403; the dense mesh bare stent segment 40 is formed by weaving the first braided wires 403; the mesh wires of the waist stent segment 30 are second braided wires 301; the waist stent segment 30 is formed by weaving the second braided wires 301; the wire diameter of the first braided wires 403 is less than the wire diameter of the second braided wires 301; and both the first braided wires 403 and the second braided wires 301 are shape memory alloy wires, preferably nickel-titanium wires.

Since the covering film 20 is provided on the waist stent segment 30, the second braided wire 301 of the waist stent segment 30 is in contact with the airway inner wall 70 through the covering film 20, while the first braided wire 403 of the dense mesh bare stent segment 40 is in direct contact with the airway inner wall 70, easily stimulating the airway inner wall 70. Therefore, by reducing the wire diameter of the first braided wire 403 of the dense mesh bare stent segment 40, the pressure of the single first braided wire 403 on the airway inner wall 70 is reduced, and the dense mesh bare stent segment 40 is made softer, thereby reducing the stimulation of the airway inner wall 70 by the dense mesh bare stent segment 40 in direct contact with the airway inner wall 70, and preventing the growth of granulation tissue 90.

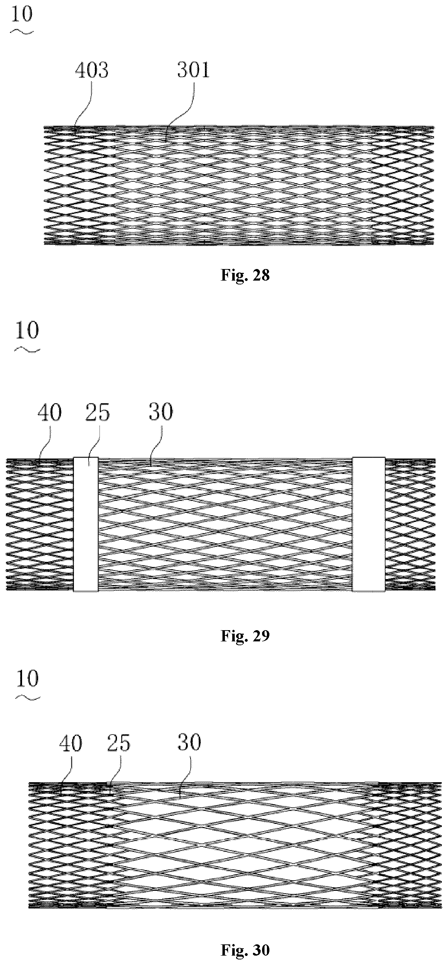

As shown in Fig. 28, the number of the first braided wires 403 of the dense mesh bare stent segment 40 is set to be equal to the number of the second braided wires 301 of the waist stent segment 30, and by setting the wire diameter of the first braided wires 403 to be less than the wire diameter of the mesh wires of the second braided wires 301, thereby reducing the pressure of a single first braided wire 403 on the airway inner wall 70, reducing the pressure of the dense mesh bare stent segment 40 as a whole on the airway inner wall 70, it is also possible to effectively reduce the stimulation of the airway inner wall 70 by the airway stent and prevent the proliferation of granulation tissue 90.

Further, as shown in conjunction with Figs. 29 and 30, the number of the first braided wires 403 of the dense mesh bare stent segment 40 is set to be greater than the number of the second braided wires 301 of the waist stent segment 30.

Since the wire diameter of the first braided wire 403 is less than the wire diameter of the second braided wire 301, the radial support force of the single first braided wire 403 is less than the radial support force of the second braided wire 301. To further increase the anchoring force between the dense mesh bare stent segment 40 and the inner wall of the airway 70, the number of the first braided wires 403 is set to be larger than the number of the second braided wires 301. That is, by reducing the wire diameter of the individual mesh wires and increasing the number of mesh wires, the pressure of the dense mesh bare stent segment 40 against the airway inner wall 70 is made more uniform. While ensuring the integral anchoring force of the dense mesh bare stent segment 40, the pressure of a single mesh wire on the airway inner wall 70 is reduced, and the pressure per unit area of the airway inner wall 70 is reduced, so that the stimulation of the airway inner wall 70 becomes smaller.

In this embodiment, the dense mesh bare stent segment 40 and the waist stent segment 30 are formed separately, and the dense mesh bare stent segment 40 and the waist stent segment 30 are fixed by a connecting portion 25.

As shown in Fig. 29, when the dense mesh bare stent segment 40 is formed separately from the waist stent segment 30, and the dense mesh bare stent segment 40 is fixed to the waist stent segment 30 by the connecting portion 25. The connecting portion 25 is a connecting film, and the dense mesh bare stent segment 40 and the waist stent segment 30 are respectively connected and fixed with the connecting film so that the dense mesh bare stent segment 40 and the waist stent segment 30 are indirectly fixedly connected via the connecting film.

In other embodiments, as shown in Fig. 30, the connecting portion 25 may also be a weld, and the first braided wire 403 of the dense mesh bare stent segment 40 may also be fixedly connected to the second braided wire 301 of the waist stent segment 30 by welding.

In other implementations, the connecting portion 25 may also be a steel sleeve (not shown), and the connecting ends of the first braided wire 403 of the dense mesh bare stent segment 40 and the second braided wire 301 of the waist stent segment 30 are fixed through a steel sleeve.

It should be noted that the above-mentioned connection modes of the dense mesh bare stent segment 40 and the waist stent segment 30 formed in sections in the present application are merely examples, and any connection mode capable of connecting the dense mesh bare stent segment 40 and the waist stent segment 30 can be applied in the present application.

Concerning the separately formed dense mesh bare stent segment 40 and the waist stent segment 30, after being respectively made and formed, they are connected via a connecting portion 25, the forming method is simple and the structure design is flexible; in the design and production process, different mesh wire diameters, mesh wire numbers and weaving methods can be selected according to actual needs, with a high degree of diversity, and can be adapted to the needs of patients with various conditions.

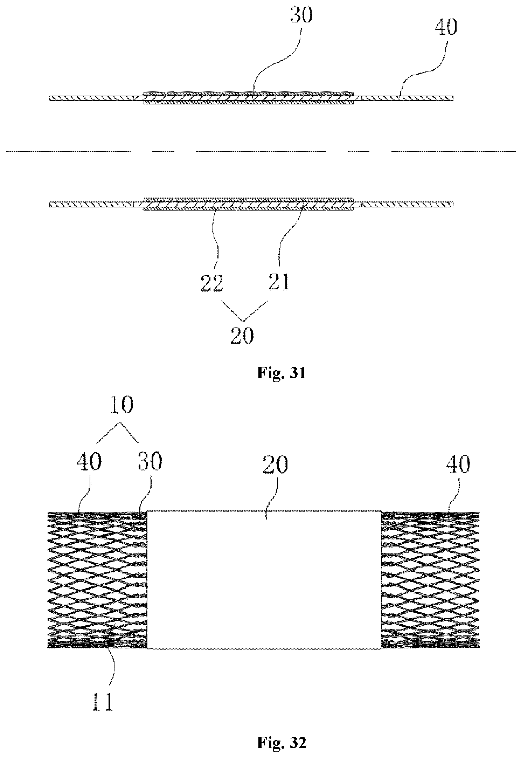

As shown in Figs. 26 and 31, the covering film 20 includes an outer film 22 provided on the outer surface of the waist stent segment 30, and the covering film 20 may further include an inner film 21 provided on the inner surface of the waist stent segment 30, and the inner film 21 and the outer film 22 are fixed in combination through the mesh hole 11 of the waist stent segment 30.

Specifically, to further prevent the malignant tumor 80 in the airway from growing into the stent body 10, resulting in restenosis of the inner wall of the airway 70 or difficulty in removal of the stent body 10, the embodiment is provided with a biocompatible polymer covering film 20 on the outer surface of the waist stent segment 30 for further preventing the malignant tumor 80 from growing into the stent body 10.

The material of the covering film 20 can be selected from PET, PTFE, ePTFE, silicone, polyurethane, etc. and the thickness of the covering film 20 is adjusted according to the size of the braided wire of the stent body 10 and the inner and outer diameters of the stent body 10, which is not limited in the present application.

To enhance the bonding strength between the covering film 20 and the stent body 10 and further prevent the tumor 80 from ingrowth, the covering film 20 of the present application further includes an inner film 21 provided on the inner surface of the waist stent segment 30, and since the stent body 10 has the mesh hole 11, the inner film 21 and the outer film 22 can be bonded and fixed through the mesh hole 11 of the waist stent segment 30. The inner film 21 and the outer film 22 can be fixed by hot melting or by bonding, and the materials of the inner film 21 and the outer film 22 can be selected to be the same or different.

In summary, according to the airway stent of the present application, first, the covering film 20 is provided on the waist stent segment 30, and the covering film 20 can reduce the stimulation of the airway inner wall 70 caused by the waist stent segment 30, and at the same time, the covering film 20 can also prevent the tumor 80 from growing into the airway stent.

Second, a dense mesh bare stent segment 40 is provided at the end portion of the waist stent segment 30, and the wire diameter of the wire of the dense mesh bare stent segment 40 is less than the wire diameter of the wire of the waist stent segment 30, reducing the pressure of a single wire against the inner wall 70 of the air passage. The present application further sets the number of the mesh wires of the dense mesh bare stent segment 40 to be greater than the number of the mesh wires of the waist stent segment 30, thereby ensuring an anchoring force between the dense mesh bare stent segment 40 and the inner wall of the airway 70, reducing the pressure received per unit area of the inner wall of the airway 70, and making the inner wall of the airway 70 less stimulated.

In addition, since both ends of the waist stent segment 30 are designed with the dense mesh bare stent segment 40, there is no retention of secretions at both ends of the airway stent. Therefore, by using the airway stent of the present application, it is possible to prevent the airway secretions from being retained on the surface of the airway stent, prevent the tumor 80 from growing inside the airway stent, reduce the stimulation of the airway inner wall 70 by the airway stent, thereby reducing the proliferation of granulation tissue 90, and avoid the occurrence of airway restenosis.

### Embodiment 8

Embodiment 8 of the present application provides an airway stent, and as shown in Figs. 32-34, the similarities between Embodiment 8 and Embodiment 7 will not be described in detail, and the difference between Embodiment 8 and Embodiment 7 is that the dense mesh bare stent segment 40 is integrally knitted with the waist stent segment 30. Specifically, the dense mesh bare stent segment 40 is knitted by a first braided wire 403, and the waist stent segment 30 is knitted by a second braided wire 301. A plurality of first braided wires 403 are intertwined to form the second braided wire 301.

The process of weaving and forming the stent body 10 includes the following steps:
First, at least two first braided wires 403 are wound around each other to prepare a second braided wire 301. The waist stent segment 30 at the waist position of the stent body 10 is braided by a plurality of prepared second braided wires 301.

Then, after the waist stent segment 30 is knitted and formed, the mesh wires that are not knitted at both ends of the waist stent segment 30 are disassembled to reform the first braided wires 403. The dense mesh bare stent segments 40 at both ends of the stent body 10 are braided by a plurality of unraveled first braided wires 403.

Finally, the knitted stent body 10 is heat-set.

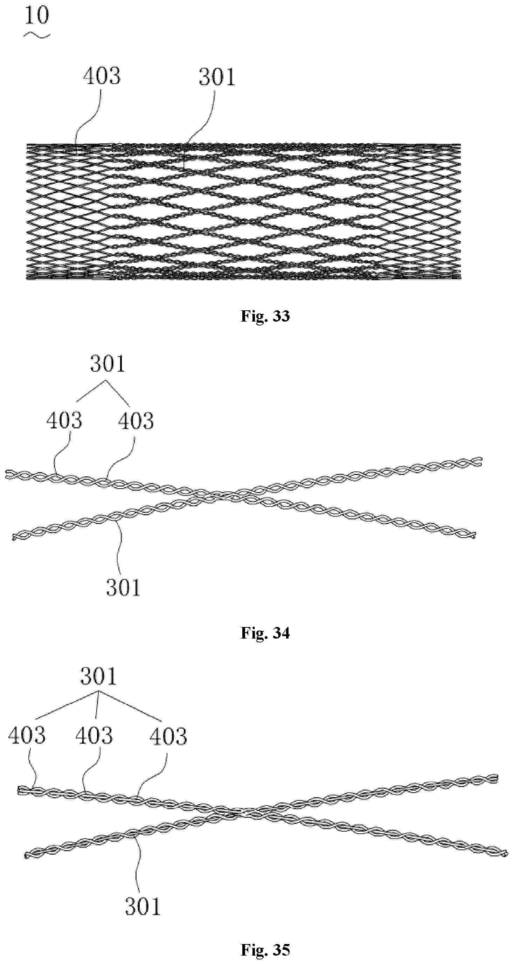

Note that the second braided wire 301 may be formed by two first braided wires 403 intertwined with each other, as shown in Fig. 35, or may be formed by three first braided wires 403 intertwined with each other, and the number of the first braided wires 403 for preparing the first braided wire is selected according to actual needs.

In addition, the stent body 10 may include a dense mesh bare stent segment 40 arranged at one end of the waist stent segment 30, and the stent body 10 may also include two dense mesh bare stent segments 40 respectively arranged at two ends of the waist stent segment 30, made according to the actual condition of the patient.

From the above, the stent body 10 obtained by integrally knitting and heat-setting has better flexibility and smoother edges; the stent body 10 has a stronger structure and higher safety.

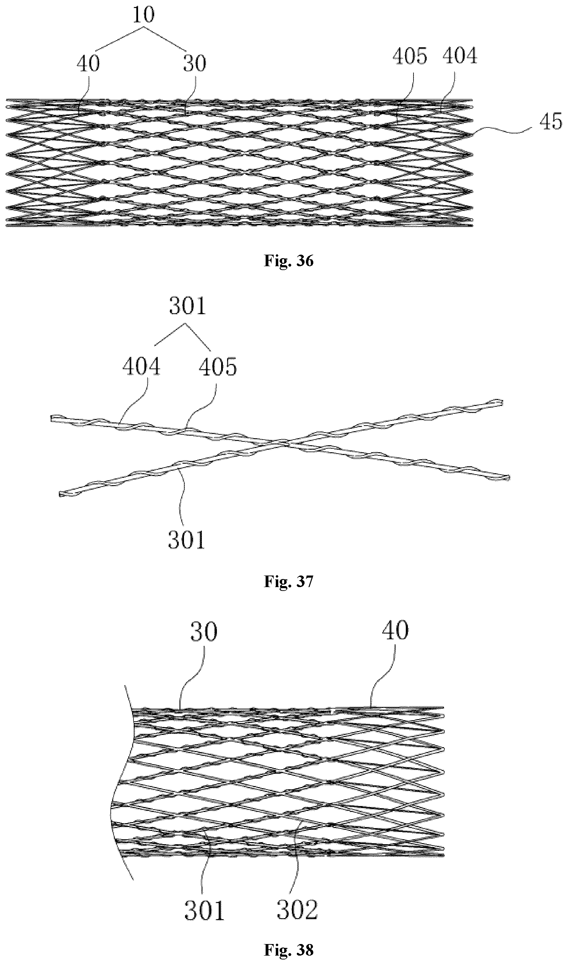

In other embodiments, as shown in Figs. 36 and 37, the first braided wire 403 includes a primary braided wire 404 and at least one secondary braided wire 405; the secondary braided wire 405 is wrapped around the primary braided wire 404 to form a second braided wire 301, the second braided wire 301 is braided to form the waist stent segment 30. Specifically, the primary braided wire 404 has a wire diameter larger than that of the secondary braided wire 405; when winding between the primary braided wire 404 and the secondary braided wire 405, the secondary braided wire 405 is coiled around the primary braided wire 404 to form a second braided wire 301.

According to this embodiment, on the one hand, the overall stability of the braided structure of the waist stent segment 30 is ensured by the provision of the primary braided wires 404, and the overall radial support of the second braided wires 301 is increased by the addition of the secondary braided wires 405. On the other hand, since the wire diameter of the secondary braided wire 405 is less than that of the primary braided wire 404, the dense mesh bare stent segment 40 formed by the mixed weaving of the primary braided wire 404 and the secondary braided wire 405 has a smaller wire diameter, the pressure of a single secondary braided wire 405 on the airway inner wall 70 is smaller, the pressure per unit area of the airway inner wall 70 is reduced, and the stimulation of the dense mesh bare stent segment 40 on the airway inner wall 70 can be further reduced.

As shown in Fig. 36, the end portion of the dense mesh bare stent segment 40 includes a mesh wire concentration point 45 formed by a plurality of first braided wires 403 converging, and when the secondary braided wire 405 is wound on the primary braided wire 404 to form the second braided wire 301, the head end of the secondary braided wire 405 can also be directly connected to the end portion of the dense mesh bare stent segment 40, thereby reducing the number of the mesh wire concentration points 45 of the dense mesh bare stent segment 40 and enhancing the strength of the single mesh wire concentration point 45, so that the anchoring force of the end portion of the dense mesh bare stent segment 40 is stronger, effectively preventing the stent body 10 from being displaced.

### Embodiment 9

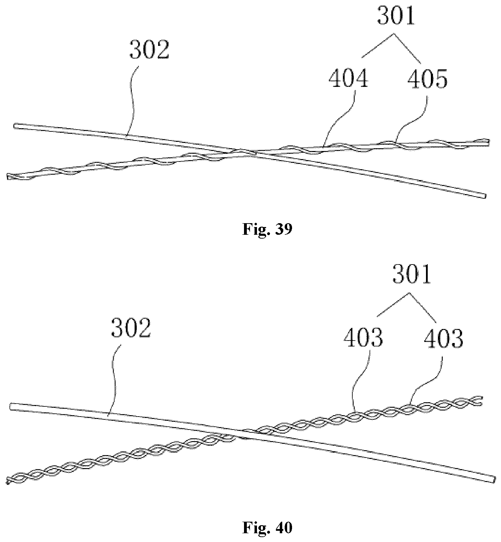

Embodiment 9 of the present application provides an airway stent, and as shown in Figs. 38-40, the similarities between embodiment 9 and embodiment 8 are not described in detail. Embodiment 9 differs from embodiment 8 in that the stent body 10 further includes at least one third braided wire 302, the waist stent segment 30 is formed by co-weaving the second braided wire 301 and the third braided wire 302, and the bare dense stent segment 40 is formed by co-weaving the first braided wire 403 and the third braided wire 302. The second braided wire 301 may be formed by weaving a plurality of first braided wires 403 having the same wire diameter, or may be formed by weaving a primary braided wire 404 and a secondary braided wire 405 having different wire diameters.

Specifically, when the stent body 10 is prepared, the radial supporting force of the stent body 10 as a whole can be adjusted by setting the wire diameter and the number of the third braided wire 302, and the pressure of the dense mesh bare stent segment 40 against the inner wall of the airway 70 can also be adjusted.

When the total number of the second braided wire 301 and the third braided wire 302 is constant, the radial support force of the entire stent body 10 increases as the yarn diameter of the third braided wire 302 increases. For example, when the wire diameter of the third braided wire 302 is selected to be larger than the wire diameter of the second braided wire 301, the radial supporting force of the stent body 10 as a whole may be increased; when the wire diameter of the third braided wire 302 is selected to be greater than the wire diameter of the first braided wire 403 and less than or equal to the wire diameter of the second braided wire 301, the radial support force of the dense mesh bare stent segment 40 can be reduced while ensuring the radial support force of the waist stent segment 30; when the wire diameter of the third braided wire 302 is selected to be less than or equal to the wire diameter of the first braided wire 403, the radial supporting force of the stent body 10 as a whole can be reduced.

In the case where the total number of the second braided wire 301 and the third braided wire 302 is constant, when the number of the third braided wire 302 is larger, the number of the second braided wire 301 is smaller, so the number of the first braided wire 403 is smaller, and the number of the mesh wires of the dense mesh bare stent segment 40 is also be smaller, and therefore, the pressure of the dense mesh bare stent segment 40 on the inner wall 70 of the air passage will be greater; and vice versa.

According to the airway stent of the embodiment, not only retention of secretions at both ends of the airway stent can be prevented, the tumor 80 can be prevented from growing into the airway stent, and granulation tissue 90 can be prevented from growing. It is also possible to flexibly adjust the wire diameter and the number of the first braided wire 403, the second braided wire 301 and the third braided wire 302, flexibly adjust the radial supporting force of the whole stent body 10, and the pressure of the dense mesh bare stent segment 40 on the inner wall of the airway 70, thereby adapting to the requirements of different patients.

The above is only preferred implementations of the present application, but the scope of protection of the present application is not limited to this. Any technical personnel familiar with the technical field of the present application can easily think of changes or replacements within the technical scope revealed by the present application, which should be covered within the scope of protection of the present application. Therefore, the scope of protection of the present application shall be subject to the scope of protection of the claim.

## Claims

1. An airway stent, comprising a stent body and a covering film provided on the stent body, wherein the stent body comprises a waist stent segment and a dense mesh bare stent segment provided at an end portion of the waist stent segment; the covering film is provided on the waist stent segment; a mesh density of the dense mesh bare stent segment is higher than a mesh density of the waist stent segment.

2. The airway stent according to claim 1, wherein the dense mesh bare stent segment comprises a single-layer dense braided mesh structure or a multi-layer braided mesh structure.

3. The airway stent according to claim 2, wherein the dense mesh bare stent segment comprises an inner layer support mesh and an outer layer support mesh; one end of the inner layer support mesh is connected to the waist stent segment, and the other end of the inner layer support mesh is connected to the outer layer support mesh; or, one end of the outer layer support mesh is connected to the waist stent segment, and the other end of the outer layer support mesh is connected to the inner layer support mesh.

4. The airway stent according to claim 3, wherein the inner layer support mesh is integrally formed with the waist stent segment; an end portion of the inner layer support mesh is turned out and folded back to form the outer layer support mesh, and the inner layer support mesh is connected to the outer layer support mesh by a folded end portion.

5. The airway stent according to claim 4, wherein the folded end portion is convex relative to the outer layer support mesh in a direction away from the stent body; the outer layer support mesh is provided with at least one convex point support portion, and the convex point support portion has a shape same as or different from that of the folded end portion.

6. The airway stent according to claim 3, wherein a sandwich film is provided between the inner layer support mesh and the outer layer support mesh.

7. The airway stent according to claim 1, wherein the covering film comprises an outer film provided on an outer surface of the waist stent segment and an inner film provided on an inner surface of the waist stent segment, the inner film and the outer film being combined and fixed through a mesh hole of the waist stent segment.

8. The airway stent according to any one of claims 1 to 7, wherein the stent body is further provided with an extension film covering an inner surface of the dense mesh bare stent segment.

9. The airway stent according to claim 1, wherein the waist stent segment is provided with a suture line for increasing connection strength between the covering film and the waist stent segment.

10. The airway stent according to claim 1, wherein a recovery line is provided on the dense mesh bare stent segment at one end of the stent body, the recovery line being threaded along an edge of the dense mesh bare stent segment.

11. An airway stent, comprising a stent body and a covering film provided on the stent body, wherein the stent body comprises a waist stent segment and a dense mesh bare stent segment provided at an end portion of the waist stent segment; the covering film is provided on the waist stent segment, and the covering film comprises an outer film provided on an outer surface of the waist stent segment and an inner film provided on an inner surface of the waist stent segment.

12. The airway stent according to claim 11, wherein the inner film and the outer film are combined and fixed through a mesh hole of the waist stent segment.

13. The airway stent according to claim 11, wherein the stent body is further provided with an extension film covering an inner surface of the dense mesh bare stent segment.

14. The airway stent according to claim 11, wherein a mesh density of the dense mesh bare stent segment is higher than a mesh density of the waist stent segment, the dense mesh bare stent segment being a single-layer dense braided mesh structure or a multi-layer braided mesh structure.

15. The airway stent according to claim 14, wherein the dense mesh bare stent segment comprises an inner layer support mesh and an outer layer support mesh; one end of the inner layer support mesh is connected to the waist stent segment, and the other end of the inner layer support mesh is connected to the outer layer support mesh; or, one end of the outer layer support mesh is connected to the waist stent segment, and the other end of the outer layer support mesh is connected to the inner layer support mesh.

16. The airway stent according to claim 15, wherein a sandwich film is provided between the inner layer support mesh and the outer layer support mesh.

17. The airway stent according to claim 15, wherein the inner layer support mesh is integrally formed with the waist stent segment; an end portion of the inner layer support mesh is turned out and folded back to form the outer layer support mesh, and the inner layer support mesh is connected to the outer layer support mesh by a folded end portion.

18. The airway stent according to claim 15, wherein the folded end portion is convex relative to the outer layer support mesh in a direction away from the stent body; the outer layer support mesh is provided with at least one convex point support portion, and the convex point support portion has a shape same as or different from that of the folded end portion.

19. The airway stent according to claim 11, wherein the waist stent segment is provided with a suture line for increasing connection strength between the covering film and the waist stent segment.

20. The airway stent according to claim 11, wherein a recovery line is provided on the dense mesh bare stent segment at one end of the stent body, the recovery line being threaded along an edge of the dense mesh bare stent segment.

21. An airway stent, comprising a stent body and a covering film provided on the stent body, wherein the stent body comprises a waist stent segment and a dense mesh bare stent segment provided at an end portion of the waist stent segment; the covering film is provided on the waist stent segment; a wire diameter of a mesh wire of the dense mesh bare stent segment is less than or equal to a wire diameter of a mesh wire of the waist stent segment.

22. The airway stent according to claim 21, wherein the number of mesh wires of the dense mesh bare stent segment is greater than the number of mesh wires of the waist stent segment.

23. The airway stent according to claim 22, wherein the dense mesh bare stent segment comprises a single-layer dense braided mesh structure or a multi-layer braided mesh structure.

24. The airway stent according to claim 23, wherein the dense mesh bare stent segment is formed separately from the waist stent segment, and the dense mesh bare stent segment is fixed to the waist stent segment by a connecting portion; the dense mesh bare stent segment is integrally knitted with the waist stent segment.

25. The airway stent according to claim 24, wherein the stent body comprises a plurality of first braided wires and a plurality of second braided wires; the dense mesh bare stent segment is braided by the first braided wires and the waist stent segment is braided by the second braided wires; a plurality of the first braided wires are intertwined to form the second braided wire.

26. The airway stent according to claim 25, wherein the first braided wire comprises a primary braided wire and at least one secondary braided wire; the secondary braided wire is wound on the primary braided wire to form the second braided wire.

27. The airway stent according to claim 25 or 26, wherein the stent body further comprises at least one third braided wire; the waist stent segment is formed by co-braiding the second braided wire and the third braided wire, and the dense mesh stent segment is formed by co-braiding the first braided wire and the third braided wire.

28. The airway stent according to claim 21, wherein the covering film comprises an outer film provided on an outer surface of the waist stent segment.

29. The airway stent according to claim 28, wherein the covering film further comprises an inner film provided on an inner surface of the waist stent segment, the inner film and the outer film being combined and fixed through a mesh hole of the waist stent segment.

30. The airway stent according to claim 21, wherein the waist stent segment is provided with a suture line for increasing connection strength between the covering film and the waist stent segment.
